# EUROPEAN PATENT APPLICATION

(11) **EP 4 552 598 A2**
(43) Date of publication of application: **14.05.2025**
(21) Application number: 24212600.1
(22) Date of filing: 13.11.2024
(51) Int. Cl.: A61B 34/30, A61B 17/00

(54) **SURGICAL ROBOTIC SYSTEM AND METHOD FOR ACCESS PORT SIZE IDENTIFICATION**

(30) Priority: 13.11.2023 US 202363598247 P; 11.10.2024 US 202418912819
(71) Applicant: Covidien LP, Mansfield, MA 02048 (US)
(72) Inventor: APOSTOLOPOULOS, Haralambos P., Mansfield, 02048 (US); TSCHUDY, Christopher T., Mansfield, 02048 (US); ROBERTS, Connor D., Mansfield, 02048 (US)
(74) Representative: Maschio & Soames IP Ltd

(57) **Abstract**

A surgical robotic system (10) includes a laparoscopic access port (55), which includes a cannula (102) and a top portion (106) having a diameter (X). The system also includes a surgical robotic arm (40) that has a port latch (46c), the port latch including at least one movable arm (114) for closing at least partially around the top portion of the access port. The system additionally includes a position sensor (120) for detecting a position of the at least one movable arm, and a controller (21a) for determining a type of the access port based on the detected position of the at least one movable arm.

## Description

### CROSS-REFERENCE TO RELATED APPLICATIONS

This application claims the benefit of, and priority to, U.S. Provisional Patent Application Serial No. 63/598,247 filed on November 13, 2023, and U.S. Patent Application Serial No. 18/912,819, filed on October 11, 2024. The entire contents of the foregoing applications are incorporated by reference herein.

### BACKGROUND

Surgical robotic systems are currently being used in a variety of surgical procedures, including minimally invasive surgical procedures. Some surgical robotic systems include a surgeon console providing for teleoperative control of a surgical robotic arm and a surgical instrument having an end effector (e.g., forceps or grasping instrument) coupled to and actuated by the robotic arm. In operation, the robotic arm is moved to a position over a patient and then guides the surgical instrument into a small incision via a surgical access port, i.e., trocar, or a natural orifice of a patient to position the end effector at a work site within the patient's body. A laparoscopic camera, which is also held by one of the robotic arms, is inserted into the patient to image the surgical site in a similar manner, i.e., through the access port.

The access ports are inserted into the patient and are attached to the robotic arm, e.g., via a clamp. Between the robot arm and the access port, a sterilization barrier, which may be a drape, makes it difficult for certain architectures to electrically connect the access port to the robot arm. Access ports have different sizes and lengths depending on the instrument size as well as the patient's features. It is important to know which type of access port is attached to the robotic arm. The length of the access port defines the position of the instrument at which the instrument may be moved freely. If the instrument's end effector is moved too early, i.e., when the end effector is still inside the access port, the movement could damage the access port's cannula. Thus, there is a need to detect the type of the access port being used.

### SUMMARY

According to one embodiment of the present disclosure a surgical robotic system is disclosed. The system includes a laparoscopic access port including a cannula and a top portion having a diameter, and a surgical robotic arm including a port latch. The port latch includes at least one movable arm for closing at least partially around the top portion of the access port. The system also includes a position sensor for detecting a position of the at least one movable arm and a controller for determining a type of the access port based on the detected position of the at least one movable arm.

Implementations of the above embodiment may include one or more of the following features. According to one aspect of the above embodiment, the type of the access port may include at least one of a diameter of the cannula, a length of the cannula, or a material of the cannula. The position sensor may be an encoder. The port latch may further include a stationary arm. The surgical robotic system may further include a sterile drape and a boot disposed behind the at least one movable arm. The boot may be configured to hold the sterile drape such that the sterile drape covers a portion of the surgical robotic arm. The boot may be formed from a flexible polymer. The sterile drape may be formed from a polyamide. The controller may access a database storing a plurality of diameters and corresponding access port types. The controller may determine a diameter of the plurality of diameters corresponding to the detected position of the at least one movable arm. The controller may also determine the type of the access post based on the determined diameter and load calibration parameters based on the determined diameter.

According to another embodiment of the present disclosure, a method for determining a type of access port in a surgical robotic system is disclosed. The method includes engaging a laparoscopic access port having a cannula and a top portion having a diameter, with a port latch of a surgical robotic arm, the port latch having a movable arm for closing at least partially around the top portion of the access port. The method also includes detecting a position of the at least one movable arm using a position sensor. The method further includes determining, by a controller, a type of the access port based on the detected position of the at least one movable arm.

Implementations of the above embodiment may include one or more of the following features. According to one aspect of the above embodiment, the method may also include determining, by the controller, that the type of the access port is one of a diameter of the cannula, a length of the cannula, or a material of the cannula. Detecting the position of the movable arm may further include using an encoder as the position sensor to detect the position of the at least one movable arm. The method may additionally include engaging a stationary arm of the port latch with the top portion of the access port in conjunction with the movable arm. The method may also include securing a sterile drape with a boot disposed behind the at least one movable arm such that the sterile drape covers a portion of the surgical robotic arm. The boot may be formed from a flexible polymer. The sterile drape may be formed from a polyamide. The method may also include accessing, by the controller, a database storing a plurality of diameters and corresponding access port types. The method may further include determining, by the controller, a diameter from the plurality of diameters corresponding to the detected position of the at least one movable arm. The method may additionally include determining, by the controller, the type of the access port based on the determined diameter and loading calibration parameters for the access port based on the determined diameter.

### BRIEF DESCRIPTION OF THE DRAWINGS

Various embodiments of the present disclosure are described herein with reference to the drawings wherein:
FIG. 1 is a perspective view of a surgical robotic system including a control tower, a console, and one or more surgical robotic arms each disposed on a mobile cart according to an embodiment of the present disclosure;
FIG. 2 is a perspective view of a surgical robotic arm of the surgical robotic system of FIG. 1 according to an embodiment of the present disclosure;
FIG. 3 is a perspective view of a mobile cart having a setup arm with the surgical robotic arm of the surgical robotic system of FIG. 1 according to an embodiment of the present disclosure;
FIG. 4 is a schematic diagram of a computer architecture of the surgical robotic system of FIG. 1 according to an embodiment of the present disclosure;
FIG. 5 is a plan schematic view of the surgical robotic system of FIG. 1 positioned about a surgical table according to an embodiment of the present disclosure;
FIG. 6 is a schematic diagram of a system for determining phases of a surgical procedure according to an embodiment of the present disclosure;
FIG. 7 is a side view of an access port according to an embodiment of the present disclosure;
FIG. 8 is a side view of a port latch and the access port according to an embodiment of the present disclosure;
FIG. 9 is a top perspective view of the port latch including a first arm and a second arm, in which the second arm is movable relative to the first arm, according to an embodiment of the present disclosure; and
FIG. 10 shows a flow chart of a method for identifying access port size according to an embodiment of the present disclosure.

### DETAILED DESCRIPTION

Embodiments of the presently disclosed surgical robotic system are described in detail with reference to the drawings, in which like reference numerals designate identical or corresponding elements in each of the several views. As used herein the term "coupled to" denotes a connection between components, which may be direct or indirect(i.e., through one or more components) and may be electronic, electrical, mechanical, or combinations thereof.

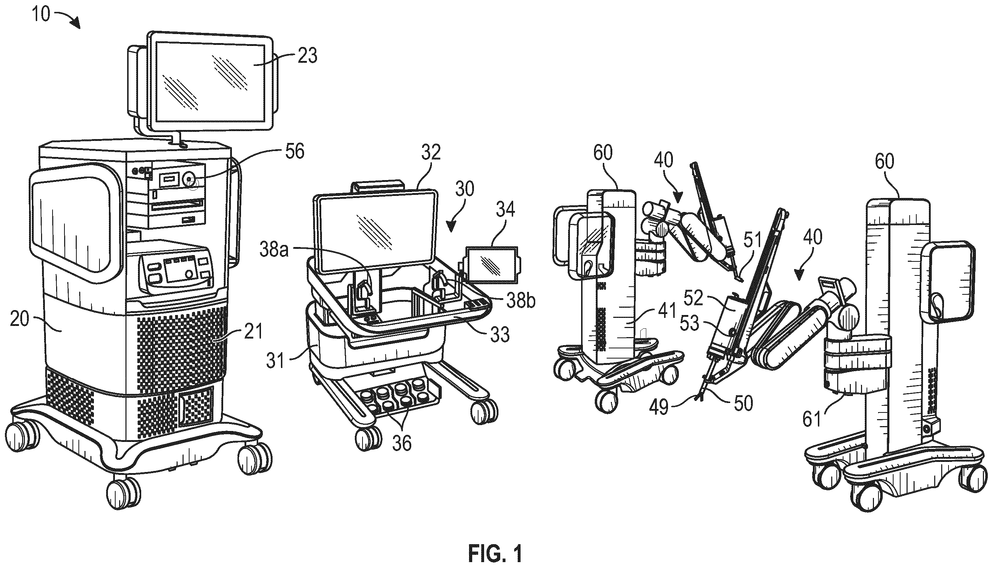

With reference to FIG. 1, a surgical robotic system 10 includes a control tower 20, which is connected to all the components of the surgical robotic system 10 including a surgeon console 30 and one or more mobile carts 60. Each of the mobile carts 60 includes a robotic arm 40 having a surgical instrument 50 removably coupled thereto. The robotic arms 40 also couple to the mobile carts 60. The robotic system 10 may include any number of mobile carts 60 and/or robotic arms 40.

The surgical instrument 50 is configured for use during minimally invasive surgical procedures. In embodiments, the surgical instrument 50 may be configured for open surgical procedures. In further embodiments, the surgical instrument 50 may be an electrosurgical or ultrasonic instrument, such as a forceps configured to seal tissue by compressing tissue between jaw members and applying electrosurgical current or ultrasonic vibrations via an ultrasonic transducer to the tissue. In yet further embodiments, the surgical instrument 50 may be a surgical stapler including a pair of jaws configured to grasp and clamp tissue while deploying a plurality of tissue fasteners, e.g., staples, and cutting stapled tissue. In yet further embodiments, the surgical instrument 50 may be a surgical clip applier including a pair of jaws configured apply a surgical clip onto tissue. The system also includes an electrosurgical generator configured to output electrosurgical (e.g., monopolar or bipolar) or ultrasonic energy in a variety of operating modes, such as coagulation, cutting, sealing, etc. Suitable generators include a Valleylab^{™} FT10 Energy Platform available from Medtronic of Minneapolis, MN.

One of the robotic arms 40 may include a laparoscopic camera 51 configured to capture video of the surgical site. The laparoscopic camera 51 may be a stereoscopic camera configured to capture two side-by-side (i.e., left and right) images of the surgical site to produce a video stream of the surgical scene. The laparoscopic camera 51 is coupled to an image processing device 56, which may be disposed within the control tower 20. The image processing device 56 may be any computing device configured to receive the video feed from the laparoscopic camera 51 and output the processed video stream.

The surgeon console 30 includes a first, i.e., surgeon, screen 32, which displays a video feed of the surgical site provided by camera 51 of the surgical instrument 50 disposed on the robotic arm 40, and a second screen 34, which displays a user interface for controlling the surgical robotic system 10. The first screen 32 and second screen 34 may be touchscreens allowing for displaying various graphical user inputs.

The surgeon console 30 also includes a plurality of user interface devices, such as foot pedals 36 and a pair of hand controllers 38a and 38b which are used by a user to remotely control robotic arms 40. The surgeon console further includes an armrest 33 used to support clinician's arms while operating the hand controllers 38a and 38b.

The control tower 20 includes a screen 23, which may be a touchscreen, and outputs on the graphical user interfaces (GUIs). The control tower 20 also acts as an interface between the surgeon console 30 and one or more robotic arms 40. In particular, the control tower 20 is configured to control the robotic arms 40, such as to move the robotic arms 40 and the corresponding surgical instrument 50, based on a set of programmable instructions and/or input commands from the surgeon console 30, in such a way that robotic arms 40 and the surgical instrument 50 execute a desired movement sequence in response to input from the foot pedals 36 and the hand controllers 38a and 38b. The foot pedals 36 may be used to enable and lock the hand controllers 38a and 38b, repositioning camera movement and electrosurgical activation/deactivation. In particular, the foot pedals 36 may be used to perform a clutching action on the hand controllers 38a and 38b. Clutching is initiated by pressing one of the foot pedals 36, which disconnects (i.e., prevents movement inputs) the hand controllers 38a and/or 38b from the robotic arm 40 and corresponding instrument 50 or camera 51 attached thereto. This allows the user to reposition the hand controllers 38a and 38b without moving the robotic arm(s) 40 and the instrument 50 and/or camera 51. This is useful when reaching control boundaries of the surgical space.

Each of the control tower 20, the surgeon console 30, and the robotic arm 40 includes a respective computer 21, 31, 41. The computers 21, 31, 41 are interconnected to each other using any suitable communication network based on wired or wireless communication protocols. The term "network," whether plural or singular, as used herein, denotes a data network, including, but not limited to, the Internet, Intranet, a wide area network, or a local area network, and without limitation as to the full scope of the definition of communication networks as encompassed by the present disclosure. Suitable protocols include, but are not limited to, transmission control protocol/intemet protocol (TCP/IP), datagram protocol/intemet protocol (UDP/IP), and/or datagram congestion control protocol (DC). Wireless communication may be achieved via one or more wireless configurations, e.g., radio frequency, optical, Wi-Fi, Bluetooth (an open wireless protocol for exchanging data over short distances, using short length radio waves, from fixed and mobile devices, creating personal area networks (PANs), ZigBee^{®} (a specification for a suite of high level communication protocols using small, low-power digital radios based on the IEEE 122.15.4-1203 standard for wireless personal area networks (WPANs)).

The computers 21, 31, 41 may include any suitable processor (not shown) operably connected to a memory (not shown), which may include one or more of volatile, non-volatile, magnetic, optical, or electrical media, such as read-only memory (ROM), random access memory (RAM), electrically-erasable programmable ROM (EEPROM), non-volatile RAM (NVRAM), or flash memory. The processor may be any suitable processor (e.g., control circuit) adapted to perform the operations, calculations, and/or set of instructions described in the present disclosure including, but not limited to, a hardware processor, a field programmable gate array (FPGA), a digital signal processor (DSP), a central processing unit (CPU), a microprocessor, and combinations thereof. Those skilled in the art will appreciate that the processor may be substituted for by using any logic processor (e.g., control circuit) adapted to execute algorithms, calculations, and/or set of instructions described herein.

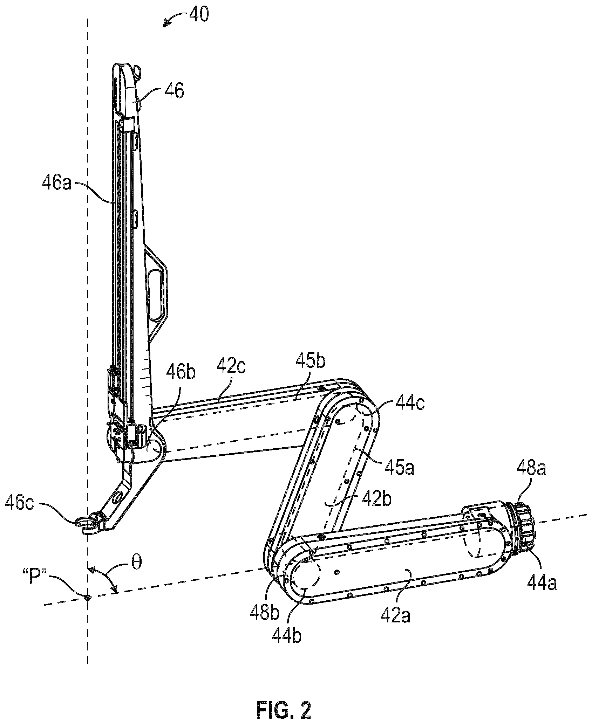

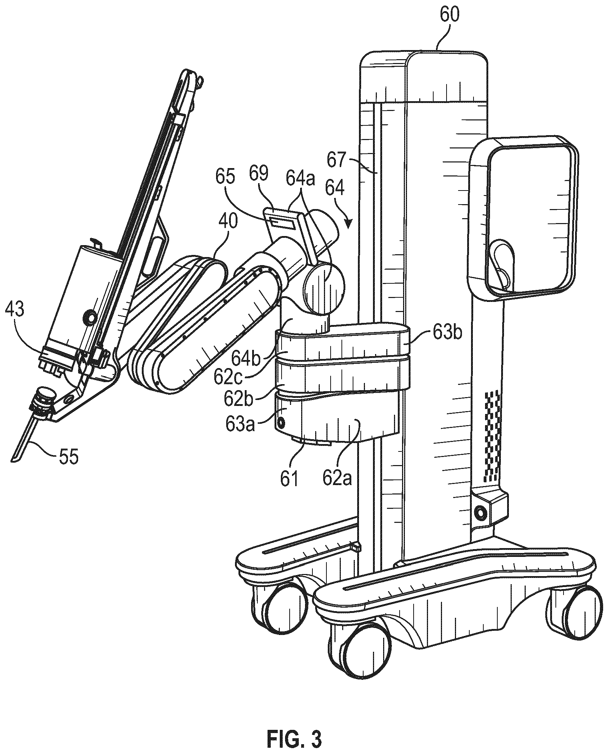

With reference to FIG. 2, each of the robotic arms 40 may include a plurality of links 42a, 42b, 42c, which are interconnected at joints 44a, 44b, 44c, respectively. Other configurations of links and joints may be utilized as known by those skilled in the art. The joint 44a is configured to secure the robotic arm 40 to the mobile cart 60 and defines a first longitudinal axis. With reference to FIG. 3, the mobile cart 60 includes a lift 67 and a setup arm 61, which provides a base for mounting the robotic arm 40. The lift 67 allows for vertical movement of the setup arm 61. The mobile cart 60 also includes a screen 69 for displaying information pertaining to the robotic arm 40. In embodiments, the robotic arm 40 may include any type and/or number of joints.

The setup arm 61 includes a first link 62a, a second link 62b, and a third link 62c, which provide for lateral maneuverability of the robotic arm 40. The links 62a, 62b, 62c are interconnected at joints 63a and 63b, each of which may include an actuator (not shown) for rotating the links 62b and 62b relative to each other and the link 62c. In particular, the links 62a, 62b, 62c are movable in their corresponding lateral planes that are parallel to each other, thereby allowing for extension of the robotic arm 40 relative to the patient (e.g., surgical table). In embodiments, the robotic arm 40 may be coupled to the surgical table (not shown). The setup arm 61 includes controls 65 for adjusting movement of the links 62a, 62b, 62c as well as the lift 67. In embodiments, the setup arm 61 may include any type and/or number of joints.

The third link 62c may include a rotatable base 64 having two degrees of freedom. In particular, the rotatable base 64 includes a first actuator 64a and a second actuator 64b. The first actuator 64a is rotatable about a first stationary arm axis which is perpendicular to a plane defined by the third link 62c and the second actuator 64b is rotatable about a second stationary arm axis which is transverse to the first stationary arm axis. The first and second actuators 64a and 64b allow for full three-dimensional orientation of the robotic arm 40.

The actuator 48b of the joint 44b is coupled to the joint 44c via the belt 45a, and the joint 44c is in turn coupled to the joint 44b via the belt 45b. Joint 44c may include a transfer case coupling the belts 45a and 45b, such that the actuator 48b is configured to rotate each of the links 42b, 42c and a holder 46 relative to each other. More specifically, links 42b, 42c, and the holder 46 are passively coupled to the actuator 48b which enforces rotation about a pivot point "P" which lies at an intersection of the first axis defined by the link 42a and the second axis defined by the holder 46. In other words, the pivot point "P" is a remote center of motion (RCM) for the robotic arm 40. Thus, the actuator 48b controls the angle θ between the first and second axes allowing for orientation of the surgical instrument 50. Due to the interlinking of the links 42a, 42b, 42c, and the holder 46 via the belts 45a and 45b, the angles between the links 42a, 42b, 42c, and the holder 46 are also adjusted to achieve the desired angle θ. In embodiments, some or all of the joints 44a, 44b, 44c may include an actuator to obviate the need for mechanical linkages.

The joints 44a and 44b include an actuator 48a and 48b configured to drive the joints 44a, 44b, 44c relative to each other through a series of belts 45a and 45b or other mechanical linkages such as a drive rod, a cable, or a lever and the like. In particular, the actuator 48a is configured to rotate the robotic arm 40 about a longitudinal axis defined by the link 42a.

With reference to FIG. 2, the holder 46 defines a second longitudinal axis and configured to receive an instrument drive unit (IDU) 52 (FIG. 1). The IDU 52 is configured to couple to an actuation mechanism of the surgical instrument 50 and the camera 51 and is configured to move (e.g., rotate) and actuate the instrument 50 and/or the camera 51. IDU 52 transfers actuation forces from its actuators to the surgical instrument 50 to actuate components an end effector 49 of the surgical instrument 50. The holder 46 includes a sliding mechanism 46a, which is configured to move the IDU 52 along the second longitudinal axis defined by the holder 46. The holder 46 also includes a joint 46b, which rotates the holder 46 relative to the link 42c. During laparoscopic procedures, the instrument 50 may be inserted through a laparoscopic access port 55 (FIG. 3) held by the holder 46. The holder 46 also includes a port latch 46c for securing the access port 55 to the holder 46 (FIG. 2).

The robotic arm 40 also includes a plurality of manual override buttons 53 (FIG. 1) disposed on the IDU 52 and the setup arm 61, which may be used in a manual mode. The user may press one or more of the buttons 53 to move the component associated with the button 53.

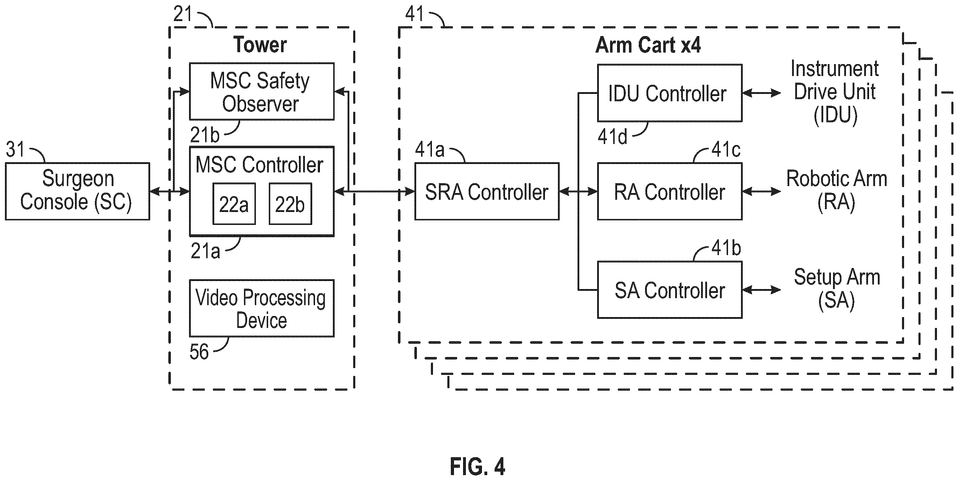

With reference to FIG. 4, each of the computers 21, 31, 41 of the surgical robotic system 10 may include a plurality of controllers, which may be embodied in hardware and/or software. The computer 21 of the control tower 20 includes a controller 21a and safety observer 21b. The controller 21a receives data from the computer 31 of the surgeon console 30 about the current position and/or orientation of the hand controllers 38a and 38b and the state of the foot pedals 36 and other buttons. The controller 21a processes these input positions to determine desired drive commands for each j oint of the robotic arm 40 and/or the IDU 52 and communicates these to the computer 41 of the robotic arm 40. The controller 21a also receives the actual joint angles measured by encoders of the actuators 48a and 48b and uses this information to determine force feedback commands that are transmitted back to the computer 31 of the surgeon console 30 to provide haptic feedback through the hand controllers 38a and 38b. The safety observer 21b performs validity checks on the data going into and out of the controller 21a and notifies a system fault handler if errors in the data transmission are detected to place the computer 21 and/or the surgical robotic system 10 into a safe state.

The controller 21a is coupled to a storage 22a, which may be non-transitory computer-readable medium configured to store any suitable computer data, such as software instructions executable by the controller 21a. The controller 21a also includes transitory memory 22b for loading instructions and other computer readable data during execution of the instructions. In embodiments, other controllers of the system 10 include similar configurations.

The computer 41 includes a plurality of controllers, namely, a main cart controller 41a, a setup arm controller 41b, a robotic arm controller 41c, and an instrument drive unit (IDU) controller 41d. The main cart controller 41a receives and processes joint commands from the controller 21a of the computer 21 and communicates them to the setup arm controller 41b, the robotic arm controller 41c, and the IDU controller 41d. The main cart controller 41a also manages instrument exchanges and the overall state of the mobile cart 60, the robotic arm 40, and the IDU 52. The main cart controller 41a also communicates actual joint angles back to the controller 21a.

Each of joints 63a and 63b and the rotatable base 64 of the setup arm 61 are passive joints (i.e., no actuators are present therein) allowing for manual adjustment thereof by a user. The joints 63a and 63b and the rotatable base 64 include brakes that are disengaged by the user to configure the setup arm 61. The setup arm controller 41b monitors slippage of each of joints 63a and 63b and the rotatable base 64 of the setup arm 61, when brakes are engaged or can be freely moved by the operator when brakes are disengaged, but do not impact controls of other joints. The robotic arm controller 41c controls each joint 44a and 44b of the robotic arm 40 and calculates desired motor torques required for gravity compensation, friction compensation, and closed loop position control of the robotic arm 40. The robotic arm controller 41c calculates a movement command based on the calculated torque. The calculated motor commands are then communicated to one or more of the actuators 48a and 48b in the robotic arm 40. The actual joint positions are then transmitted by the actuators 48a and 48b back to the robotic arm controller 41c.

The IDU controller 41d receives desired joint angles for the surgical instrument 50, such as wrist and jaw angles, and computes desired currents for the motors in the IDU 52. The IDU controller 41d calculates actual angles based on the motor positions and transmits the actual angles back to the main cart controller 41a.

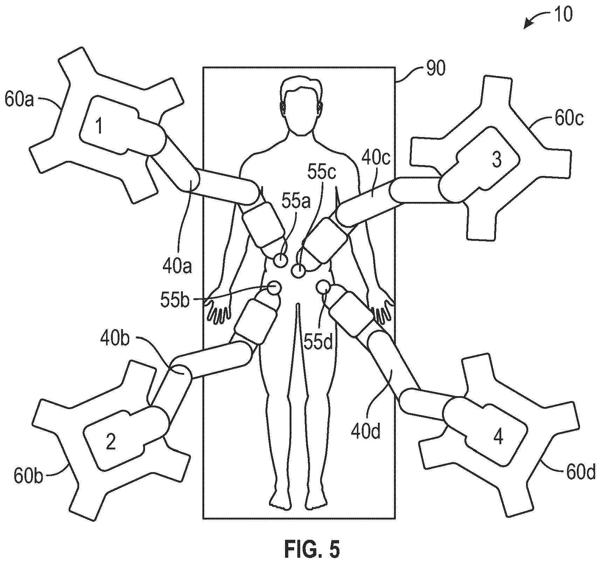

With reference to FIG. 5, the surgical robotic system 10 is set up around a surgical table 90. The system 10 includes mobile carts 60a-d, which may be numbered "1" through "4." During setup, each of the carts 60a-d are positioned around the surgical table 90. Position and orientation of the carts 60a-d depends on a plurality of factors, such as placement of a plurality of access ports 55a-d, which in turn, depends on the surgery being performed. Once the port placement is determined, the access ports 55a-d are inserted into the patient, and carts 60a-d are positioned to insert instruments 50 and the laparoscopic camera 51 into corresponding ports 55a-d.

During use, each of the robotic arms 40a-d is attached to one of the access ports 55a-d that is inserted into the patient by attaching the latch 46c (FIG. 2) to the access port 55 (FIG. 3). The IDU 52 is attached to the holder 46, followed by the SIM 43 being attached to a distal portion of the IDU 52. Thereafter, the instrument 50 is attached to the SIM 43. The instrument 50 is then inserted through the access port 55 by moving the IDU 52 along the holder 46. The SIM 43 includes a plurality of drive shafts configured to transmit rotation of individual motors of the IDU 52 to the instrument 50 thereby actuating the instrument 50. In addition, the SIM 43 provides a sterile barrier between the instrument 50 and the other components of robotic arm 40, including the IDU 52. The SIM 43 is also configured to secure a sterile drape (not shown) to the IDU 52.

A surgical procedure may include multiple phases, and each phase may include one or more surgical actions. As used herein, the term "phase" represents a surgical event that is composed of a series of steps (e.g., closure). A "surgical action" may include an incision, a compression, a stapling, a clipping, a suturing, a cauterization, a sealing, or any other such actions performed to complete a phase in the surgical procedure. A "step" refers to the completion of a named surgical objective (e.g., hemostasis). During each step, certain surgical instruments 50 (e.g., forceps) are used to achieve a specific objective by performing one or more surgical actions.

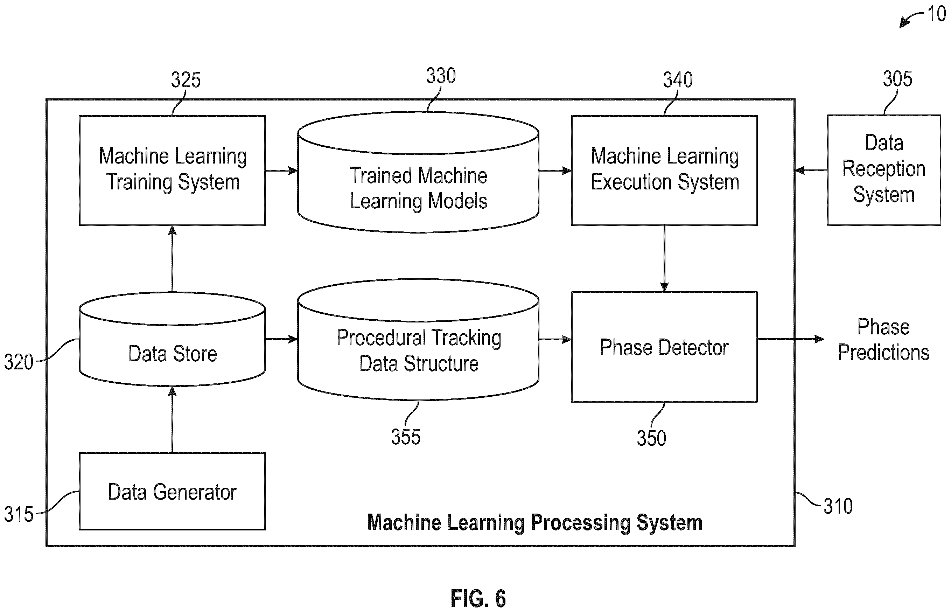

With reference to FIG. 6, the surgical robotic system 10 may include a machine learning (ML) processing system 310 that processes the surgical data using one or more ML models to identify one or more features, such as surgical phase, instrument, anatomical structure, etc., in the surgical data. The ML processing system 310 includes a ML training system 325, which may be a separate device (e.g., server) that stores its output as one or more trained ML models 330. The ML models 330 are accessible by a ML execution system 340. The ML execution system 340 may be separate from the ML training system 325, namely, devices that "train" the models are separate from devices that "infer," i.e., perform real-time processing of surgical data using the trained ML models 330.

System 10 includes a data reception system 305 that collects surgical data, including the video data and surgical instrumentation data. The data reception system 305 can include one or more devices (e.g., one or more user devices and/or servers) located within and/or associated with a surgical operating room and/or control center. The data reception system 305 can receive surgical data in real-time, i.e., as the surgical procedure is being performed.

The ML processing system 310, in some examples, may further include a data generator 315 to generate simulated surgical data, such as a set of virtual images, or record the video data from the image processing device 56, to train the ML models 330 as well as other sources of data, e.g., user input, arm movement, etc. Data generator 315 can access (read/write) a data store 320 to record data, including multiple images and/or multiple videos.

The ML processing system 310 also includes a phase detector 350 that uses the ML models to identify a phase within the surgical procedure. Phase detector 350 uses a particular procedural tracking data structure 355 from a list of procedural tracking data structures. Phase detector 350 selects the procedural tracking data structure 355 based on the type of surgical procedure that is being performed. In one or more examples, the type of surgical procedure is predetermined or input by user. The procedural tracking data structure 355 identifies a set of potential phases that may correspond to a part of the specific type of surgical procedure.

In some examples, the procedural tracking data structure 355 may be a graph that includes a set of nodes and a set of edges, with each node corresponding to a potential phase. The edges may provide directional connections between nodes that indicate (via the direction) an expected order during which the phases will be encountered throughout an iteration of the surgical procedure. The procedural tracking data structure 355 may include one or more branching nodes that feed to multiple next nodes and/or may include one or more points of divergence and/or convergence between the nodes. In some instances, a phase indicates a procedural action (e.g., surgical action) that is being performed or has been performed and/or indicates a combination of actions that have been performed. In some instances, a phase relates to a biological state of a patient undergoing a surgical procedure. For example, the biological state may indicate a complication (e.g., blood clots, clogged arteries/veins, etc.), pre-condition (e.g., lesions, polyps, etc.). In some examples, the ML models 330 are trained to detect an "abnormal condition," such as hemorrhaging, arrhythmias, blood vessel abnormality, etc.

The phase detector 350 outputs the phase prediction associated with a portion of the video data that is analyzed by the ML processing system 310. The phase prediction is associated with the portion of the video data by identifying a start time and an end time of the portion of the video that is analyzed by the ML execution system 340. The phase prediction that is output may include an identity of a surgical phase as detected by the phase detector 350 based on the output of the ML execution system 340. Further, the phase prediction, in one or more examples, may include identities of the structures (e.g., instrument, anatomy, etc.) that are identified by the ML execution system 340 in the portion of the video that is analyzed. The phase prediction may also include a confidence score of the prediction. Other examples may include various other types of information in the phase prediction that is output. The predicted phase may be used by the controller 21a to determine when to perform access port identification process as described below.

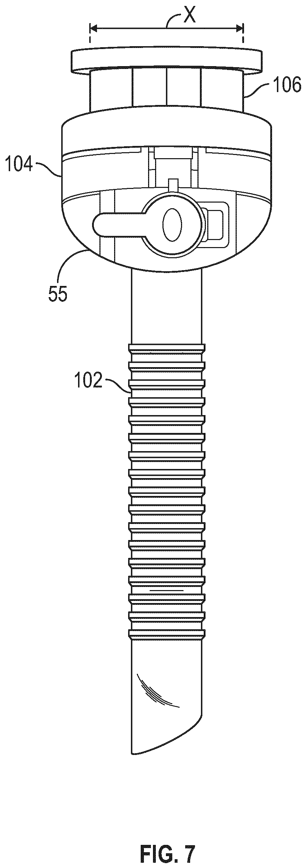

With reference to FIG. 7, the access port 55 includes a cannula 102 extending from a funnel-shaped housing 104 having one or more seals (not shown) disposed therein. The access port 55 also includes a top portion 106 having a diameter "X." Various types of access ports 55 may have corresponding, i.e., unique, diameters "X," which allow for identification of the access port 55 based on the diameter "X" of the top portion 106.

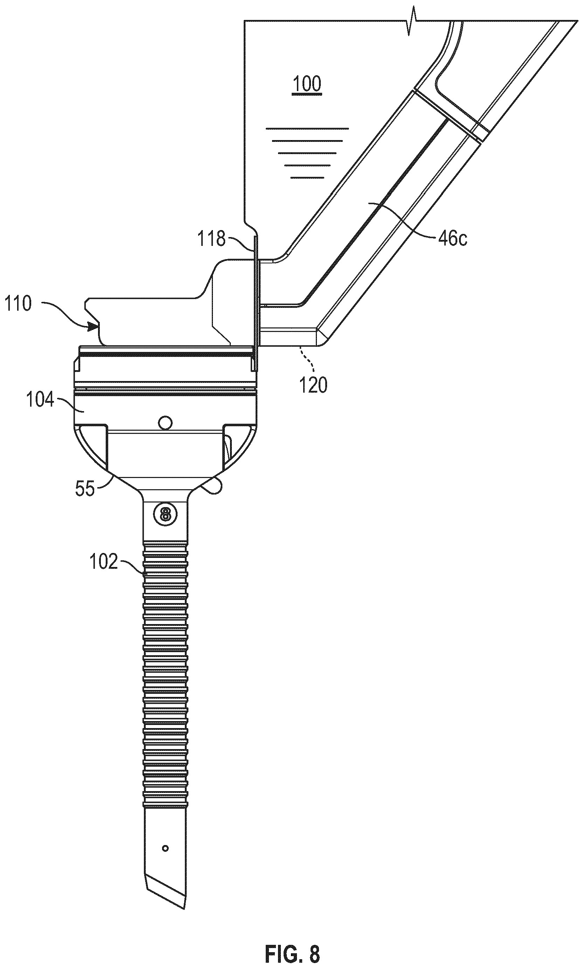

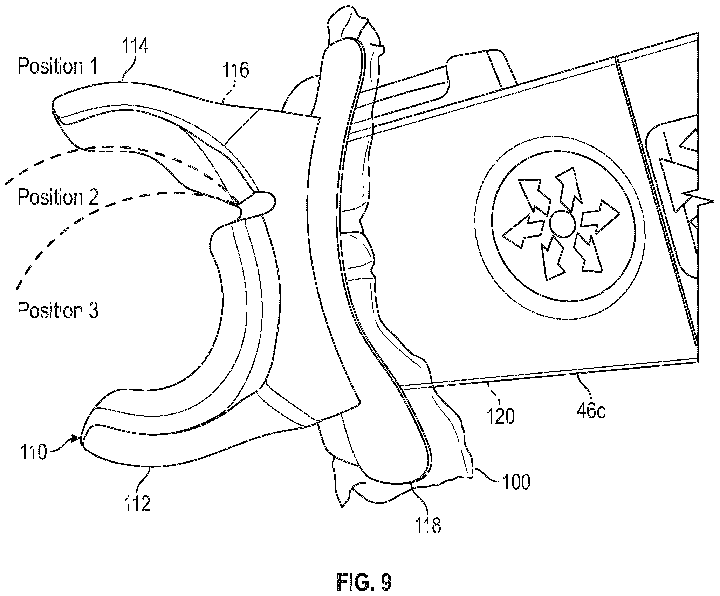

With reference to FIG. 8, the port latch 46c secures the access port 55 to the holder 46 via an arm assembly 110 configured to capture the top portion 106 of the access port 55. The arm assembly 110 may be disposed at an end of the port latch 46c. As shown, the arm assembly 110 includes a first (e.g., stationary) arm 112 and a second (e.g., movable) arm 114 (FIG. 9). In aspects, either one or both of the first and second arms 112 and 114 may be movable relative to each other. A boot 118 is disposed behind the arm assembly 110. The boot 118 may be formed from a flexible material, such as a flexible polymer, or may be comprised of any suitable material capable of accommodating, i.e., flexing and stretching, in response to a positional change of the second arm 114. A sterile drape 100 is disposed behind the boot 118, which is used to cover the robotic arm 40, including the holder 46 and a portion of the port latch 46c. During use, the sterile drape 100 is placed over the robotic arm 40 including the arm assembly 110 and the boot 118 is then placed over the sterile drape 100 and the arm assembly 110. The drape 110 is also formed from a conformable material, such as polyamide, such that the drape 110 conforms to the movable portions of the arm assembly 110 and the boot 118.

With reference to FIG. 9, the arm assembly 110 includes a mechanism 116 configured to close the arm assembly 110 around the top portion 106 of the access port 55. The mechanism 116 (not explicitly shown) may use a ratcheting motion in order to cause the second arm 114 to close to a variety of specific positions, such that a specific position of the second arm 114 captures the top portion 106 of a corresponding access port 55 between the second arm 114 and the first arm 112. While three positions (e.g., "Position 1," "Position 2," and "Position 3") of the second arm 114 are shown, any number of positions are contemplated to accommodate different access ports 55 having top portions 106 of different diameters "X."

The robotic arm 40 includes a position sensor 120 (not explicitly shown) disposed adjacent to the port latch 46c or the arm assembly 110, e.g., underneath, attached to, or in the port latch 46c or the arm assembly 110. The position sensor 120 may be disposed in any suitable manner to detect a position of the second arm 114. The position sensor may be a magnetic sensor (e.g., Hall Effect sensor), an encoder, a potentiometer, a limit switch, or any other sensor suitable for detecting the position of the second arm 114. Detection of the position of the second arm 114 allows for identification of the diameter "X" of the top portion 106 of the access port 55, and by extension the access port 55.

In aspects, the controller 21a is coupled to the position sensor 120 and is configured to receive the sensor signal corresponding to the detected position of the second arm 114. The controller 21a activates the position sensor 120 in response to a user command or automatically, in response to detection of the coupling of the access port 55 to the latch 46c. In the absence of the access port 55, the arm assembly 110 may remain fully open, i.e., in an initial position not corresponding to a diameter "X" of any access port 55. Therefore, the position sensor 120 would not produce a sensor signal, i.e., signal would be 0, and this value may be used to indicate that the access port 55 is absent. Conversely, any reading by the position sensor 120 may be used to indicate the access port 55 is present and the controller 21a may then enable operation of the position detection process to determine the type of the access port 55 based on the position of the second arm 114.

The controller 21a receives position sensor signals from the position sensor 120 and then determines the type of the access port 55 based on the detected position of the second arm 114. The controller 21a may store a database (e.g., storage 22a) including a plurality of positions of the second arm 114 and diameters of the top portion 106 of the access port 55 based on the position of the arms 112 and 114, as well as different types of access ports 55 corresponding to the diameters. Types of access ports 55 may include model numbers, dimensions (including diameters, lengths, and materials of the cannula 102), and the like. In embodiments, the database may be stored in a remote storage location (e.g., cloud) accessible by the controller 21a. In particular, the controller 21a identifies the type of the access port 55 based on the received position signal. In particular, the position of the second arm 114 is used to determine the diameter or another dimension of the access port 55 being grasped by the arm assembly 110. The determined dimension is then used to determine the type of the access port 55 from the database of acceptable access ports 55. Once identified, the controller 21a may load parameters of the access port 55 from the database for use during operation of the system 10, e.g., calibration, operational limits for the instruments 50 used with the identified access port 55, if instrument is not compatible with port based of database and current attached instrument etc.

During preparation of a laparoscopic surgical procedure, the access ports 55a-d are inserted into the patient's body. The robotic arms 40a-d are positioned and connected to the access ports 55a-d as described above with respect to FIG. 5. Then the system 10, e.g., controller 21a, activates the corresponding position sensors 120 of the arms 40a-d. The position sensor 120 detects the position of each second arm 114 and provides the value to the controller 21a. Different types of access ports 55a-d are mapped to different diameters "X," so the controller 21a can determine which access port type is connected to the robotic arms 40a-d according to the value provided by the position sensor 120. After the access port 55 is successfully identified, the position sensor 120 may be switched off.

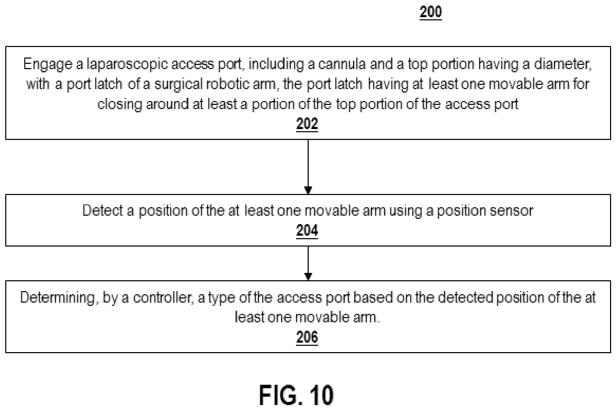

FIG. 10 shows a flow chart of a method 200 for identifying access port size. The method may be embodied as software instructions stored in a non-transitory computer readable medium and executable by a processor, e.g., controller 21a.

At step 202, the laparoscopic access port 55 is engaged with a port latch 46c of the surgical robotic arm 40. This step ensures that the access port 55 is securely held by the robotic arm 40, allowing the system 10 to interact with the access port 55 for subsequent identification and operation.

Once the access port 55 is engaged by the port latch 46c, at step 204, the system detects the position of the movable arm 114 using the position sensor 120. The position sensor 120, which may be an encoder or another suitable device, identifies the position of the movable arm 114 as it closes at least partially around the top portion 106 of the access port 55. The detected position corresponds to a specific dimension of the access port 55, such as the diameter "X" of its top portion 106.

After detecting the position of the movable arm 114, at step 206, the controller 21a determines the type of access port 55 based on the detected position of the movable arm 114. The type of access port 55 may include characteristics such as the diameter "X", length, or material of the cannula 102. This information is used by the surgical robotic system 10 to adapt its operation according to the specific access port 55, thereby providing safe and accurate operation during the surgical procedure.

It will be understood that various modifications may be made to the embodiments disclosed herein. Therefore, the above description should not be construed as limiting, but merely as exemplifications of various embodiments. Those skilled in the art will envision other modifications within the scope and spirit of the claims appended thereto.

The following examples are illustrative of the techniques described herein.

Example 1. A surgical robotic system comprising: a laparoscopic access port including a cannula and a top portion having a diameter; a surgical robotic arm including a port latch, the port latch including at least one movable arm for closing at least partially around the top portion of the access port; a position sensor for detecting a position of the at least one movable arm; and a controller for determining a type of the access port based on the detected position of the at least one movable arm.

Example 2. The surgical robotic system according to Example 1, wherein the type of the access port includes at least one of a diameter of the cannula, a length of the cannula, or a material of the cannula.

Example 3. The surgical robotic system according to Example 1, wherein the position sensor is an encoder.

Example 4. The surgical robotic system according to Example 1, wherein the port latch further includes a stationary arm.

Example 5. The surgical robotic system according to Example 1, further comprising: a sterile drape, and a boot disposed behind the at least one movable arm, wherein the boot is configured to hold the sterile drape such that the sterile drape covers a portion of the surgical robotic arm.

Example 6. The surgical robotic system according to Example 5, wherein the boot is comprised of a flexible polymer.

Example 7. The surgical robotic system according to Example 5, wherein the sterile drape is comprised of a polyamide.

Example 8. The surgical robotic system according to Example 1, wherein the controller accesses a database storing a plurality of diameters and corresponding access port types.

Example 9. The surgical robotic system according to Example 8, wherein the controller determines a diameter of the plurality of diameters corresponding to the detected position of the at least one movable arm.

Example 10. The surgical robotic system according to Example 9, wherein the controller determines the type of the access port based on the determined diameter and loads calibration parameters for the access port based on the determined diameter.

Example 11. A method for determining a type of access port in a surgical robotic system, the method comprising: engaging a laparoscopic access port, including a cannula and a top portion having a diameter, with a port latch of a surgical robotic arm, the port latch having at least one movable arm for closing at least partially around the top portion of the access port; detecting a position of the at least one movable arm using a position sensor; and determining, by a controller, a type of the access port based on the detected position of the at least one movable arm.

Example 12. The method according to Example 11, further comprising: determining, by the controller, that the type of the access port includes at least one of a diameter of the cannula, a length of the cannula, or a material of the cannula.

Example 13. The method according to Example 11, wherein detecting the position of the at least one movable arm further includes using an encoder as the position sensor to detect the position of the at least one movable arm.

Example 14. The method according to Example 11, further comprising: engaging a stationary arm of the port latch with the top portion of the access port in conjunction with the at least one movable arm.

Example 15. The method according to Example 11, further comprising: securing a sterile drape with a boot disposed behind the at least one movable arm such that the sterile drape covers a portion of the surgical robotic arm.

Example 16. The method according to Example 15, wherein the boot is comprised of a flexible polymer.

Example 17. The method according to Example 15, wherein the sterile drape is comprised of a polyamide.

Example 18. The method according to Example 11, further comprising: accessing, by the controller, a database storing a plurality of diameters and corresponding access port types.

Example 19. The method according to Example 18, further comprising: determining, by the controller, a diameter from the plurality of diameters corresponding to the detected position of the at least one movable arm.

Example 20. The method according to Example 19, further comprising: determining, by the controller, the type of the access port based on the determined diameter; and loading calibration parameters for the access port based on the determined diameter.

## Claims

1. A surgical robotic system (10) comprising:
a laparoscopic access port (55) including a cannula (102) and a top portion (106) having a diameter (X);
a surgical robotic arm (40) including a port latch (46c), the port latch including at least one movable arm (114) for closing at least partially around the top portion of the access port;
a position sensor (120) for detecting a position of the at least one movable arm; and
a controller (21a) for determining a type of the access port based on the detected position of the at least one movable arm.

2. The surgical robotic system according to claim 1, wherein the type of the access port includes at least one of a diameter of the cannula, a length of the cannula, or a material of the cannula.

3. The surgical robotic system according to any preceding claim, wherein the position sensor is an encoder.

4. The surgical robotic system according to any preceding claim, wherein the port latch further includes a stationary arm (112).

5. The surgical robotic system according to any preceding claim, further comprising:
a sterile drape (100), and
a boot (118) disposed behind the at least one movable arm, wherein the boot is configured to hold the sterile drape such that the sterile drape covers a portion of the surgical robotic arm.

6. The surgical robotic system according to claim 5, wherein the boot is comprised of a flexible polymer.

7. The surgical robotic system according to claim 5, wherein the sterile drape is comprised of a polyamide.

8. The surgical robotic system according to any preceding claim, wherein the controller accesses a database storing a plurality of diameters and corresponding access port types.

9. The surgical robotic system according to claim 8, wherein the controller determines a diameter of the plurality of diameters corresponding to the detected position of the at least one movable arm.

10. The surgical robotic system according to claim 9, wherein the controller determines the type of the access port based on the determined diameter and loads calibration parameters for the access port based on the determined diameter.

11. A method (200) for determining a type of access port (55) in a surgical robotic system (! 0), the method comprising:
engaging (202) a laparoscopic access port (55), including a cannula (102) and a top portion (106) having a diameter (X), with a port latch (46c) of a surgical robotic arm (40), the port latch having at least one movable arm (114) for closing at least partially around the top portion of the access port;
detecting (204) a position of the at least one movable arm using a position sensor (120); and
determining (206), by a controller (21a), a type of the access port based on the detected position of the at least one movable arm.

12. The method according to claim 11, further comprising:
determining, by the controller, that the type of the access port includes at least one of a diameter of the cannula, a length of the cannula, or a material of the cannula.

13. The method according to any one of claim 11 or 12, further comprising:
engaging a stationary arm of the port latch with the top portion of the access port in conjunction with the at least one movable arm.

14. The method according to any one of claims 11-13, further comprising:
securing a sterile drape with a boot disposed behind the at least one movable arm such that the sterile drape covers a portion of the surgical robotic arm.

15. The method according to any one of claims 11-14, further comprising:
accessing, by the controller, a database storing a plurality of diameters and corresponding access port types;
determining, by the controller, a diameter from the plurality of diameters corresponding to the detected position of the at least one movable arm;
determining, by the controller, the type of the access port based on the determined diameter; and
loading calibration parameters for the access port based on the determined diameter.
